# EUROPEAN PATENT APPLICATION

(11) **EP 4 335 558 A1**
(43) Date of publication of application: **13.03.2024**
(21) Application number: 22194643.7
(22) Date of filing: 08.09.2022
(51) Int. Cl.: B06B 1/06

(54) **ACOUSTIC DEVICE AND METHOD OF MANUFACTURING**

(71) Applicant: Nederlandse Organisatie voor toegepast-natuurwetenschappelijk Onderzoek TNO, 2595 DA 's-Gravenhage (NL)
(72) Inventor: Fillinger, Laurent, 2595 DA 's-Gravenhage (NL); Peters, Laurent Christiaasn Johannes Maria, 2595 DA 's-Gravenhage (NL); Gelinck, Gerwin Hermanus, 2595 DA 's-Gravenhage (NL)
(74) Representative: V.O.

(57) **Abstract**

An acoustic device (1000) comprises a sheet (100) having a contact surface (11) for contacting a medium (M) and transceiving acoustic signals (A) via the contact surface (11). The sheet has a piezoelectric layer (20) comprising piezoelectric material (20m) configured to transduce the acoustic signals (A) via the contact surface (11). A backing layer (30) is acoustically interconnected to a backside (Sb) of the piezoelectric layer (20), opposite the contact surface (11). The backing layer (30) comprises at least two sublayers (31,32) with different materials (31m,32m). The first sublayer (31) comprises a first material (31m) having a Young's modulus less than that of the piezoelectric material (20m). The second sublayer (32) comprises a second material (32m) having a density higher than that of the first material (31m). At least some of the first material (31m) is arranged between the second material (32m) and the piezoelectric material (20m).

## Description

### TECHNICAL FIELD AND BACKGROUND

The present disclosure relates to an acoustic device comprising a sheet for transducing acoustic signals; and method of manufacturing such acoustic device.

Acoustic devices capable of transducing relatively low frequencies may be useful for reaching relatively large penetration depth, e.g., cardiac/liver imaging or obstetric ultrasound. Furthermore, acoustic device having relatively large bandwidth may provide shorter acoustic signals which may improve (axial) resolutions. However, in regular ultrasound transducer designs there are limited options to optimize the frequency response for the application. Lower frequency applications typically require thicker layers of the piezo material. This is particularly problematic in an acoustic device comprising a flexible transducer array, where a larger thickness negatively affects the mechanical flexibility of a transducer. Thicker piezoelectric films also require larger poling voltages, which can be problematic. Maximization of the transducer bandwidth typically involve backing materials which also affect the flexibility of the transducer.

There is yet a desire for improving the frequency response of a relatively thin and/or flexible acoustic transducer sheet, e.g. in terms of center frequency and frequency bandwidth, while minimally affecting the flexibility and/or peak efficiency.

### SUMMARY

Aspects of the present disclosure relate to an acoustic device comprising a sheet, which is preferably relatively thin and/or flexible. The sheet has a contact surface on a front side of the sheet for contacting a medium and transceiving (transmitting and/or receiving) acoustic signals to and/or from the medium via the contact surface. The sheet further has a piezoelectric layer comprising piezoelectric material configured to transduce (generate and/or measure) the acoustic signals via the contact surface, i.e. frontside. The sheet further has a backing layer acoustically interconnected to a backside of the piezoelectric layer, opposite the contact surface. The backing layer comprises at least a first sublayer and a second sublayer. The first sublayer comprises a first material having a Young's modulus less than that of the piezoelectric material. The second sublayer comprises a second material having a density higher than that of the first material. At least some of the first material is arranged between the second material and the piezoelectric material.

Without being bound by theory, the inventors find that the backing layer comprising a combination of a relatively dense (heavy) second sublayer, connected to the piezoelectric layer via a relatively flexible (soft) first sublayer, may act as a mass-spring oscillator. Advantageously, the added mass may allow to shift the resonance frequency of the piezo-material (from λ/2 toward λ/4), without requiring a corresponding increase in total thickness of the sheet. The intermediate flexible material can be used to further control the resonant response of the transducer while having only limited effect on the flexibility of the sheet. Additionally, by patterning one or both layers, mass and/or flexibility may be disposed in the specific positions, where needed, which may locally improve the effect and allow further lowering of the bending stiffness. Accordingly, the present teachings may allow for the optimization of the frequency response of a fully flexible ultrasound transducer in terms of center frequency and frequency bandwidth without negatively affecting the transducers flexibility or peak efficiency. This allows the transducer designs to reach lower frequencies (useful for applications requiring a large penetration depth, e.g. cardiac/liver imaging or obstetric ultrasound) or reach higher bandwidths (and therefore axial resolutions) than would otherwise be possible.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A illustrates an acoustic device comprising a sheet;
FIG 1B illustrates part of the sheet contacting a medium for transceiving acoustic signals;
FIG 2A illustrates a perspective view of stack of layers forming part of a sheet;
FIG 2B illustrates a cross-section view of the stack of layers contacting a medium for transceiving acoustic signals
FIGs 3A and 3B illustrate parts of a sheet and stack comprising a structured piezoelectric layer;
FIGs 4A and 4B illustrate stacks comprising further structuring of the backing layer;
FIGs 5A and 5B illustrate stacks including an acoustic matching layer and additional foil between the piezoelectric layer and further sublayers of the backing layer;
FIGs 6 and 7 illustrate simulations for various structures and thicknesses of sublayers in the backing layer.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

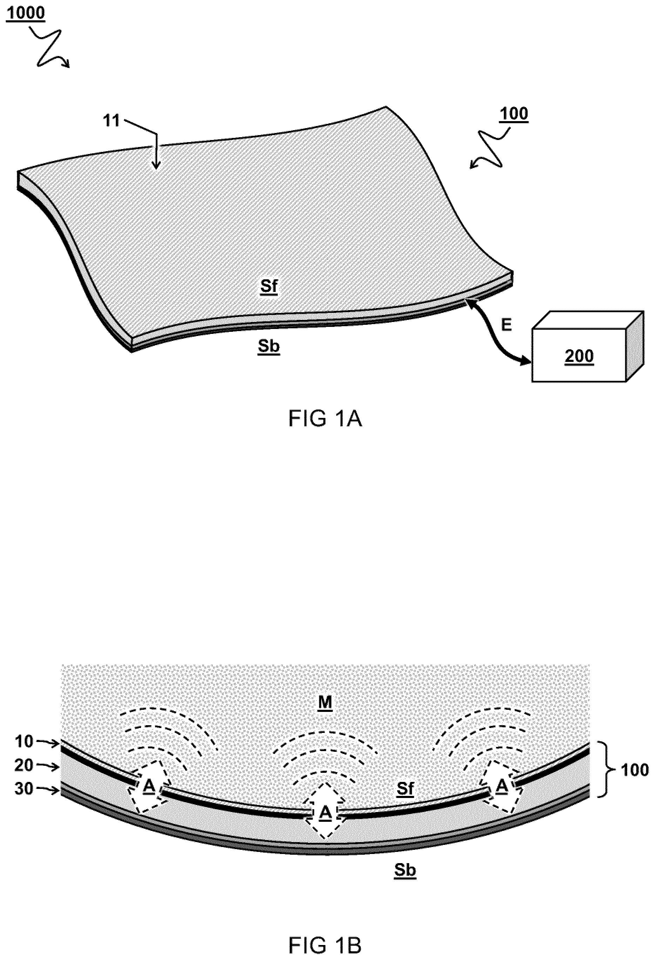

FIG 1A illustrates an acoustic device 1000 comprising a sheet 100. FIG 1B illustrate part of the sheet 100 contacting a medium "M" for transceiving acoustic signals "A". Typically, the acoustic signals "A" as described herein propagate in the medium "M" as acoustic waves. For example, the medium "M" can be a biological medium, e.g. tissue, or nonbiological medium, e.g. liquid or solid. In some embodiments, the acoustic signals "A" are ultrasound signals, e.g. with frequencies from 20 kHz up to several gigahertz. For example, the acoustic device 1000 comprises, or forms part of, an ultrasonic transducer or ultrasound device.

In some embodiments, the sheet 100 has a front layer 10. For example, the front layer 10 comprises or forms a contact surface 11 on a front side "Sf" of the sheet 100. Preferably, the contact surface 11 is suitable for contacting a medium "M". More preferably, the front layer 10 is suitable for transceiving, e.g. transmitting and/or receiving, acoustic signals "A" to and/or from the medium "M" via the contact surface 11. In other or further embodiments, the sheet 100 comprises a piezoelectric layer 20. Typically, the piezoelectric layer 20 comprises piezoelectric material 20m configured to transduce, e.g. generate and/or measure, the acoustic signals "A" via the contact surface 11, i.e. frontside "Sf". In other or further embodiments, the sheet 100 comprises a backing layer 30. Preferably, the backing layer 30 is acoustically interconnected to a backside "Sb" of the piezoelectric layer 20, opposite the contact surface 11.

In some embodiments, the sheet 100 comprises a two-dimensional array of acoustic transducers, e.g. formed by parts of the piezoelectric layer 20 sandwiched between respective electrode layers. In a preferred embodiment, the sheet is a flexible sheet. Most preferably, the sheet 100 including all layers (e.g. 10,20,30) is sufficiently flexible to allow bending of the sheet at, or below, an allowable radius without breaking and/or losing essential functionality, e.g. without breaking any of the acoustic transducers, electric wiring, structural integrity of the layers, et cetera. For example, the allowable radius may be less than one meter, preferably less than fifty centimeter, more preferably less than twenty centimeter, most preferably less than ten centimeter, or even less than five centimeter. By providing the sheet more flexibility, the sheet can be directly applied to various curved surfaces of a medium "M" such as a body part, pipe, et cetera, and acoustic waves can be more efficiently applied to and/or measured from various directions into/from the medium "M".

In some embodiments, e.g. to obtain sufficient flexibility, the thickness of the sheet 100 and/or the combined thickness of the layers 10,20,30 can be relatively low, e.g. less than one centimeter, preferably less five millimeters, more preferably less than one millimeter, most preferably less than half a millimeter, e.g. between 50 - 500 µm. In other or further embodiments, various flexible materials and/or bendable layers 10,20,30 can be used, such as polymer based materials. In one embodiment, the piezoelectric layer 20 comprises a polymer based piezoelectric material 20m. For example, the piezoelectric material 20m comprises a poled film of polyvinylidene (di)fluoride (PVDF), preferably poly(vinylidene fluoride-cotrifluoroethylene) (P(CDF-TrFE) 80/20). In another or further embodiment, the backing layer 30 comprises flexible and/or patterned materials. In principle, the conductive layers such as electrodes can be relatively thin, e.g. a thin metal layer and/or wiring. Preferably, a flexible electrode is provided, e.g. a "MAM electrode" and/or electrodes comprising metals/oxides made of thermally evaporated materials such as MoCr/Al/MoCr, MoOx/Au/MoOx, MoO3/Ag/MoO3, et cetera.

In some embodiments, the acoustic device 1000 comprises a controller 200. In one embodiment, e.g. as shown, the controller 200 is separate from the sheet 100, e.g. connected or connectable by electrical wiring. It can also be envisaged that the controller 200 or control elements are partially or fully integrated as part of the sheet 100. In one embodiment, the controller 200 is configured to control generation of acoustic signals "A" by the sheet 100. For example, the controller 200 is configured to generate electrical signals E and one or more transducers comprising parts of the piezoelectric layer 20 (in the sheet 100) are configured to convert the electrical signals E into acoustic signals "A". In another or further embodiment, the controller 200 is configured to receive and/or process measurement of the acoustic signals "A" by the sheet 100. For example, one or more transducers comprising parts of the piezoelectric layer 20 (in the sheet 100) are configured to convert acoustic signals "A" into electrical signals E, and the controller 200 is configured to measure and/or process the electrical signals E.

In some embodiments, the acoustic device 1000 is configured to generate, measure, and/or process the acoustic and/or electrical signals to determine an acoustic image of the medium "M". For example, the acoustic device 1000 comprises, or forms part of, an ultrasound imaging device. Also other applications can be envisaged, such as treatment by acoustic waves, acoustic streaming, e.g. for mixing liquids, inspection of constructional elements by acoustic waves, et cetera. In one embodiment, the controller 200 comprises or accesses a (non-transitory) computer-readable medium storing instructions that, when executed, cause the acoustic device to perform operational acts as described herein.

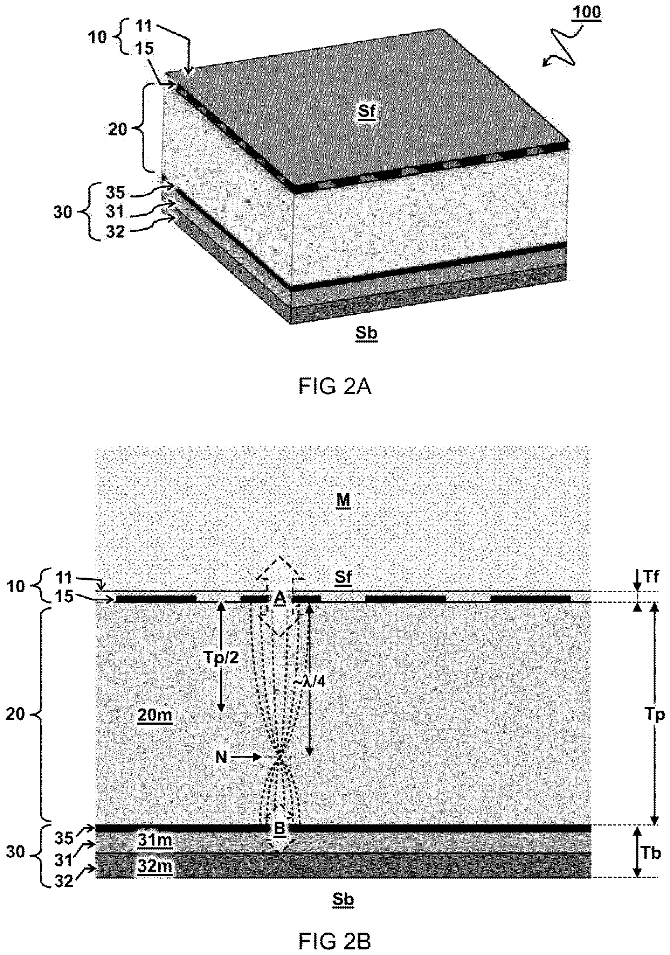

FIG 2A illustrate a perspective view of a stack of layers forming part of a sheet 100, e.g. as shown in FIGs 1A and 1B. FIG 2B illustrate a cross-section view of the stack of layers contacting a medium "M" for transceiving acoustic signals "A". As illustrated in the figures, the piezoelectric layer 20 between the front layer 10 and the backing layer 30 has a first thickness "Tp"; the backing layer 30 between the piezoelectric layer 20 and a backside "Sb" of the device 1000 has a second thickness "Tb"; and the front layer 10, between the piezoelectric layer 20 and the front side "Sf" of the device, i.e. front surface of the contact surface 11, has a third thickness "Tf". As described herein, the backing layer 30 preferably comprises at least two different sublayers 31,32 and/or substructures made of different materials 31m,32m. Preferably, at least some of the first material 31m is arranged between the second material 32m and the piezoelectric material 20m.

In some embodiments, the backing layer 30 comprises a first sublayer 31 comprising, or essentially consisting of, a first material 31m having a Young's modulus less than that of the piezoelectric material 20m and/or less than that of the second material 32m. For example, the Young's modulus of the first material 31m is less than that of the piezoelectric material 20m and/or less than that of the second material 32m (at least in the actuation direction as indicated by the arrows "B", i.e. perpendicular to the layers) by at least 10% (factor 1.1), 20% (factor 2.2), or 50% (factor 1.5), preferably at least a factor two, three, up to a factor ten, or more.

In other or further embodiments, the backing layer 30 comprises a second sublayer 32 comprising, or essentially consisting of, a second material 32m having a density higher than that of the first material 31m and/or higher than that of the piezoelectric material 20m. For example, the density (mass per unit volume of the respective material) of the second material 32m is higher than that of the piezoelectric material 20m and/or higher than that of the first material 31m by at least 10% (factor 1.1), 20% (factor 2.2), or 50% (factor 1.5), preferably at least a factor two, three, up to a factor ten, or more.

As described herein, the backing layer 30, comprising different functional sublayers 31,32, may allow to provide a sufficient effect of controlling the frequency and/or bandwidth of transducers even when the backing layer is relatively thin. Accordingly, in some embodiments, the second thickness "Tb" of the backing layer 30 is smaller than the first thickness "Tp" of the piezoelectric layer 20, e.g. by at least a factor two, three, five, up to a factor eight, ten, or more. For example, the piezoelectric layer 20 has a first thickness "Tp" between 25 - 250 µm, preferably between 50 - 150 µm. For example, the backing layer 30 has a second thickness "Tb" between 5 - 50 µm, preferably between 10 - 25 µm. In other or further embodiments, the third thickness "Tf" of the front layer 10 is smaller than the first thickness "Tp" of the piezoelectric layer 20, and preferably also smaller than the second thickness "Tb" of the backing layer 30. In one embodiment, the third thickness "Tf' is smaller than the first thickness "Tp" by at least a factor two, three, five, ten, up to a factor twenty, fifty, hundred, or more. In another or further embodiment, the third thickness "Tf" is smaller than the second thickness "Tb" by at least a factor two, three, five, up to a factor eight, ten, or more. For example, the front layer 10 has a thickness less than 50 µm, preferably less than 10 µm, or even less than 1 µm. By keeping the front layer 10 relatively thin, flexibility may be minimally affected and/or acoustic wave transmission may be optimized.

In some embodiments, e.g. as shown, the piezoelectric layer 20 comprises or essentially consists of piezoelectric material 20m which is arranged between a frontside electrode layer 15, and a backside electrode layer 35. Typically, the electrode layers comprises an electrically conductive material, such as metal; and/or may be relatively thin, e.g. having a thickness less than two micrometer, preferably less than one micrometer, e.g. between 0.1 - 0.5 µm. In one embodiment, e.g. as shown, the backside electrode layer 35 may be considered part of the backing layer 30. However, in view of the negligible thickness / mechanical function, the backside electrode layer 35 could also be considered part of the piezoelectric layer 20. Similarly, the frontside electrode layer 15 may be considered part of the front layer 10, as illustrated, or part of the piezoelectric layer 20. In one embodiment, the front layer 10 comprises at least a non-conductive layer in front of the frontside electrode layer 15 forming the contact surface 11. For example, this may be a relatively thin layer or a larger layer, including or forming an acoustic matching layer.

In some embodiments, e.g. as illustrated, the front electrode layer 15 is patterned so that electrical signals (e.g. voltage) can be selectively applied to, and/or measured from, a local area of the piezoelectric layer 20, e.g. forming a respective transducer. In other or further embodiments, e.g. as illustrated, the backside electrode layer 35 can be a continuous metal or other conductive layer acting as a common electrode. In other embodiments (not shown) the subdivision can be applied to both the frontside electrode layer 15 and the backside electrode layer 35 or both electrode layer 15,35 may be continuous layers.

As described herein, the backing layer 30 preferably comprises at least two different sublayers 31,32, i.e. comprising or essentially consisting of two different materials 31m, 32m. In some embodiments, the high density material 32m used for the second sublayer 32 may include e.g. a metal (iron, steel, copper, silver, lead, gold, platinum) or for instance ceramics. In other or further embodiments, flexible materials 31m for the first sublayer 31 may include various polymeric and organic materials, with and without fillers additives, in solid film form and/or as cellular foam.

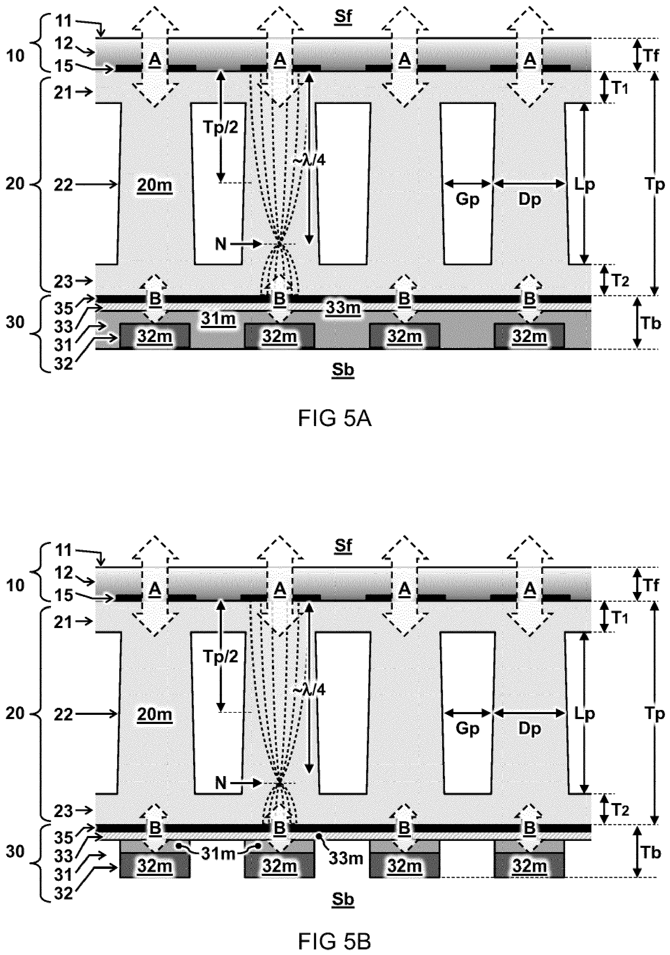

In some embodiments, the two or more sublayers 31,32 of the backing layer 30 are deposited onto, or laminated against, the piezoelectric layer 20 and/or the backside electrode layer 35, or an intermediate substrate 33, e.g. as illustrated in FIGs 5A and 5B. In other or further embodiments, the stack is built up starting with the backing layer 30, and depositing or laminating the backside electrode layer 35 and/or piezoelectric layer 20 onto the backing layer 30.

Without being bound by theory, it is observed that acoustic transducers typically provide a non-reflecting / transmissive backing layer, wherein the main resonance frequency of the piezoelectric layer may be determined by the thickness "Tp" of the piezoelectric layer 20 corresponding approximately to half a wavelength (λ/2) to form a standing wave with one node "N" midway (at Tp/2) between anti-nodes on the two opposite ends. In contrast, as illustrated in the present figures and according to some embodiments of the present backing layer 30, the mass-spring effect of the different sublayers (indicated by the acoustic coupling "B") may cause the node "N" to shift away from the midway position (at Tp/2) towards the backing layer 30. This may correspond to shifting of the resonance to lower frequencies (longer wavelengths), e.g. up to λ/4. In some embodiments, the coupling "B" may be stronger when the resonance frequency of the mass-spring system formed by the backing layer 30 (e.g. determined by the effective mass and spring constant provided by the materials and structures of the sublayers 31,32), matches the resonance frequency of piezoelectric layer 20 (e.g. determined by the thickness "Tp" and the the wave velocity in the piezoelectric material 20m). A similar effect of shifting the node "N" towards the backing layer could be achieved if the backing layer completely reflects the waves, but this would normally require the use of a much thicker high density layer, which would affect flexibility. For completeness, it is noted that the present figures illustrate the amplitude of a standing wave oscillation transverse to the propagation direction, where it will be understood that the wave may in practice predominantly oscillate in the direction of propagation.

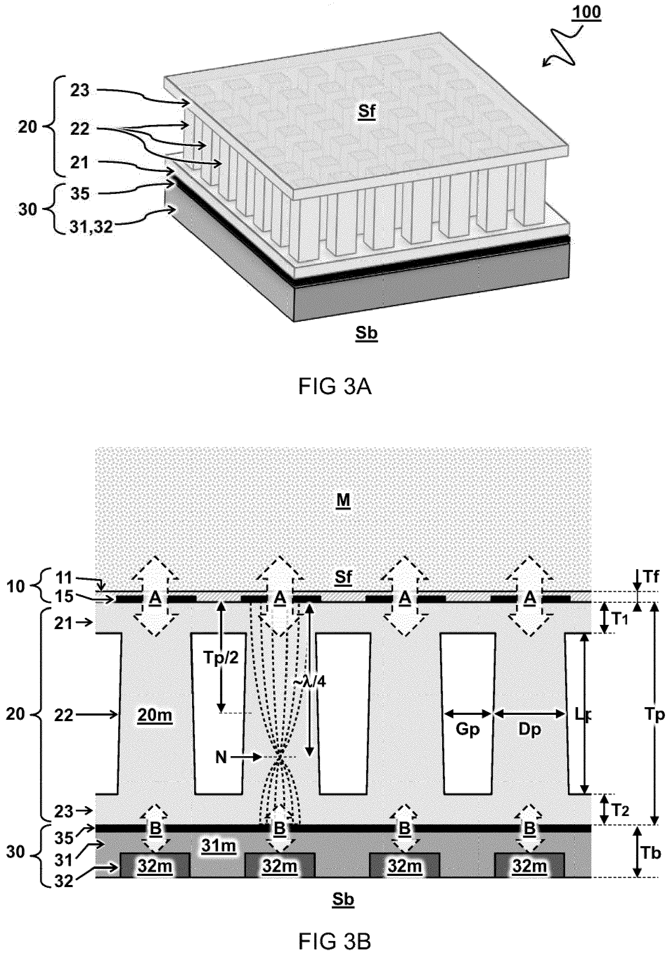

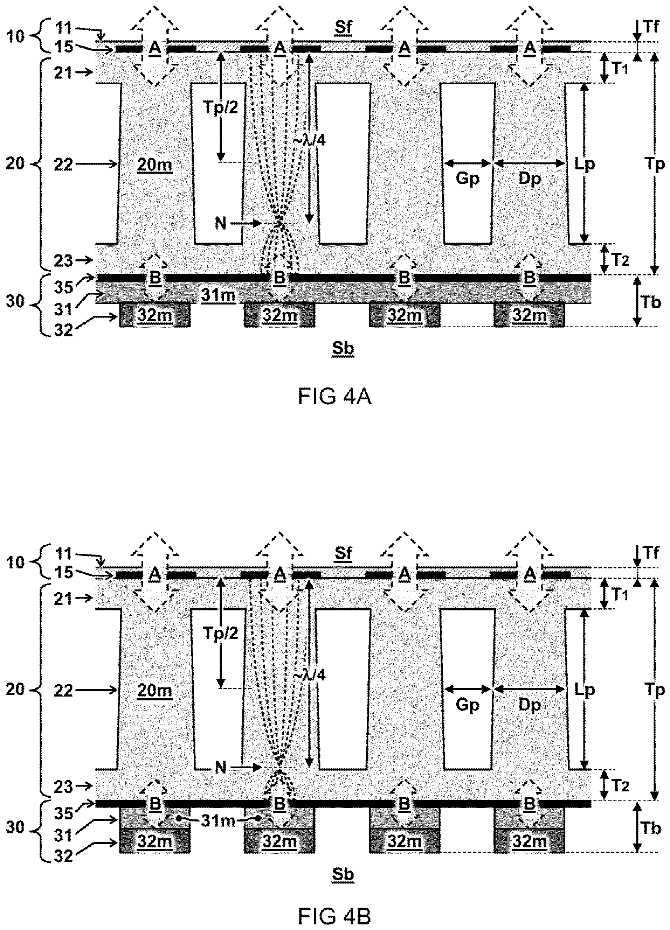

FIGs 3A and 3B illustrate parts of a sheet 100 and stack comprising a structured piezoelectric layer 20 and/or backing layer 30. FIGs 4A and 4B illustrate stacks comprising further structuring of the backing layer 30. In some embodiments, the piezoelectric layer 20 is a structured piezoelectric layer, e.g. with subdivided elements of piezoelectric material 20m forming respective acoustic transducers. For example, the subdivision of piezoelectric material 20m may correspond to the subdivision of one or both of the electrode layers 15,35. Preferably, the piezoelectric layer 20 comprises piezoelectric material 20m forming an array of pillars 22 configured to transceive acoustic signals "A" via a frontside "Sf" of the device.

In some embodiments, the pillars 22, or other subdivided elements, have a respective (maximum) diameter "Dp" (in plane of the layer) that is the same of smaller than a respective length "Lp" of the pillars (transverse to the plane of the layers, e.g. propagation direction of the acoustic signals), or other subdivided elements, e.g. smaller by at least 20% (factor 1.2), 50% (factor 1.5), up to a factor two, three, or more. The smaller the diameter compared to the length, the higher density of transducers providing relatively low frequencies. For example, the diameter "Dp" is between 10 - 100 µm, preferably between 20 - 60 µm. The diameter "Dp" does not need to be constant, e.g. can be slightly thicker on one side, as shown. To provide sufficient separation / decoupling, the (minimum) gap "Gp" may be similar to the diameter "Dp", e.g. preferably smaller. For example, the (minimum) gap "Gp" is between 5 - 50 µm, preferably between 10 - 30 µm.

In some embodiments, the piezoelectric layer 20 comprises at least one of a backside piezoelectric layer 23 or a frontside piezoelectric layer 21. Advantageously, the continuous piezoelectric layer 21 and/or 23 may allow depositing further structures and/or layers. To minimize crosscoupling between the subdivided elements 22, the continuous piezoelectric layer 21 and/or 23 preferably have an individual thickness T1 and/or T2 smaller than the length "Lp" of the subdivided elements 22, e.g. smaller by at least a factor two, three, five, up to a factor ten, or more. For example, the continuous piezoelectric layer 21 and/or 23 have a respective thickness T1,T2 between 1 - 30 µm, preferably between 5 - 20 µm.

Preferably, the array of pillars 22 is integrally formed between the backside piezoelectric layer 23 and the frontside piezoelectric layer 21. In other words, the backside piezoelectric layer 23, array of pillars 22, and frontside piezoelectric layer 21 may form a monolithic piece of material. For example, the layers 21,23 essentially consist of the same piezoelectric material 20m as the intermediate structure / pillars; and/or the piezoelectric material are melted together. In some embodiments, the piezoelectric layer 20 with pillars 22 may be manufactured according to the methods described in the earlier publication WO 2021/167446 A1. In other or further embodiments, also other structuring of the piezoelectric layer 20 can be envisaged. In some embodiments, one or both of the piezoelectric layers 21,23 can be omitted, e.g. providing pillars directly between the electrode layers 15,35. In other or further embodiments, a structured piezoelectric layer is provided by cutting or otherwise subdividing the piezoelectric layer 20 in separate islands or pillars. For example, pillar structures and/or other subdivision of the piezoelectric layer 20 can be advantageous in lowering acoustical and/or mechanical cross coupling between elements in an acoustic device.

As described herein, the backing layer 30 preferably comprises at least two different sublayers 31,32, wherein at least one of the sublayers is patterned, i.e. subdivided in elements. Preferably, the subdivision of one or more of the at least two different sublayers 31,32 corresponds to the division of the piezoelectric layer 20 forming respective transducers. For example, the patterning of the one or more sublayers corresponds to the patterning of at least one of the electrode layers 15,35 and/or corresponds to the patterning of the piezoelectric layer 20. So, it will be understood that the pattering of one or more of the sublayers, such as shown in FIGs 3B, 4A, 4B, 5A, 5B, could also be applied in a continuous piezoelectric layer 20 such as FIG 2B (not shown). The patterning can vary in vary in terms of relative width, alignment with respect to the pillar and relative pitch. Other possible variation may include designs where the heavy material 32m is fully covered by other materials (not shown).

In some embodiments, e.g. as shown in each of FIGs 3B, 4A, 4B, 5A, 5B, the second sublayer 32 comprises subdivided areas of the second material 32m. In one embodiment, e.g. as shown in FIGs 3B and 5A, the first material 31m of the first sublayer 31 extends between the subdivided areas of the second material 32m. In another or further embodiment, e.g. as shown in FIGs 4A, 4B, 5B, spacing without material is provided between the subdivided areas of the second material 32m. Also combinations are possible, or a third material can be provided between the subdivided areas of the second material 32m (not shown) and/or the second sublayer 32 can be covered by another layer (not shown). Instead of completely subdividing the second sublayer 32, it can also be envisaged to vary a layer thickness of the second material 32m, e.g. concentrating more material to some areas than other areas. In other or further embodiments, e.g. as shown in FIGs 4B and 5B, the first sublayer 31 comprises subdivided areas of the first material 31m. In one embodiment, e.g. as shown, spacing without material is provided between the subdivided areas of the first material 31m. In another or further embodiment (not shown) another material is provided between the subdivided areas of the first material 31m.

Preferably, the first sublayer 31 and/or second sublayer 32 is subdivided according to a pattern wherein more material 31m,32m of the respective sublayer 31,32 is provided at positions corresponding to positions of the piezoelectric layer 20 where respective transducers are formed. For example, material 31m,32m of the respective sublayer 31,32 is exclusively or predominantly provided (concentrated) at the positions of the pillars 22 (or similar substructure of the piezoelectric layer 20) and/or at positions of the actuation positions determined by a respective (subdivided) electrode layer 15 and/o 35.

FIGs 5A and 5B illustrate stacks including an acoustic matching layer 12 and additional foil 33 between the piezoelectric layer 20 and further sublayers 31,32 of the backing layer 30. It will be understood that either one or both of these features can be combined with any of the preceding embodiments.

In some embodiments, acoustic wave transmission to a medium "M" may be optimized by providing one or more acoustic matching layers 12, which may have a relatively large thickness "Tf', e.g. up to the second thickness "Tb", or even more. For example, the matching layer or layers may provide an acoustic impedance gradient for the acoustic waves from the piezoelectric layer / transducers to smoothly penetrate into the medium "M" and/or acoustic waves to smoothly propagate from the medium "M" to the piezoelectric layer / transducers for detection.

In other or further embodiments, acoustic behavior may be further optimized and/or manufacturing can be facilitated by providing an additional foil 33, which may be considered as part of the backing layer 30. In one embodiment, acoustic behavior can be further optimized by adjusting a thickness, material, and/or distribution of the additional layer 33. In another or further embodiment, manufacturing can be facilitated by providing foil 33 for depositing / laminating the backside electrode layer 35 and/or the other sublayers of the backing layer 30.

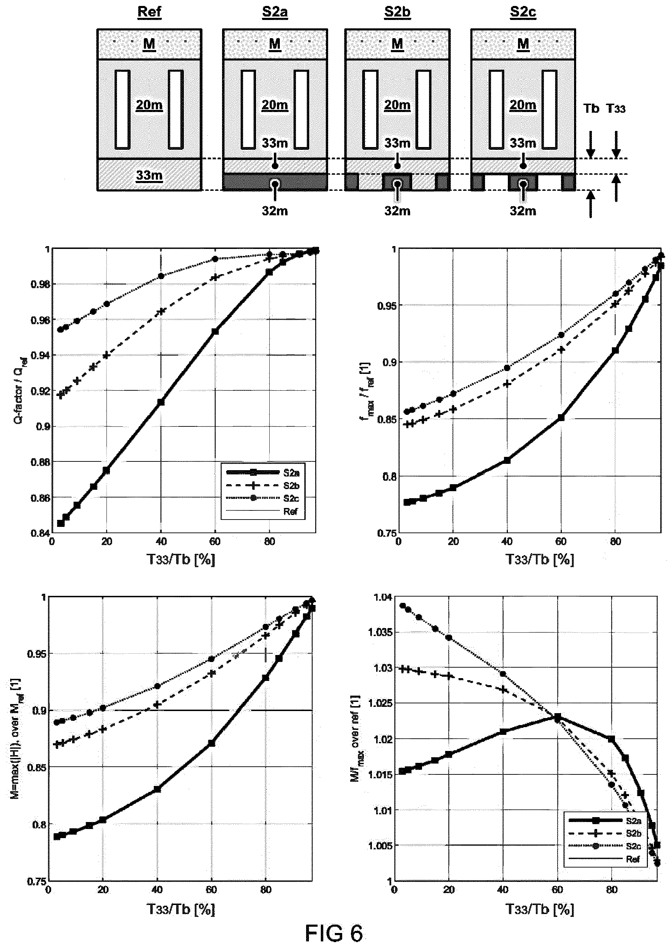

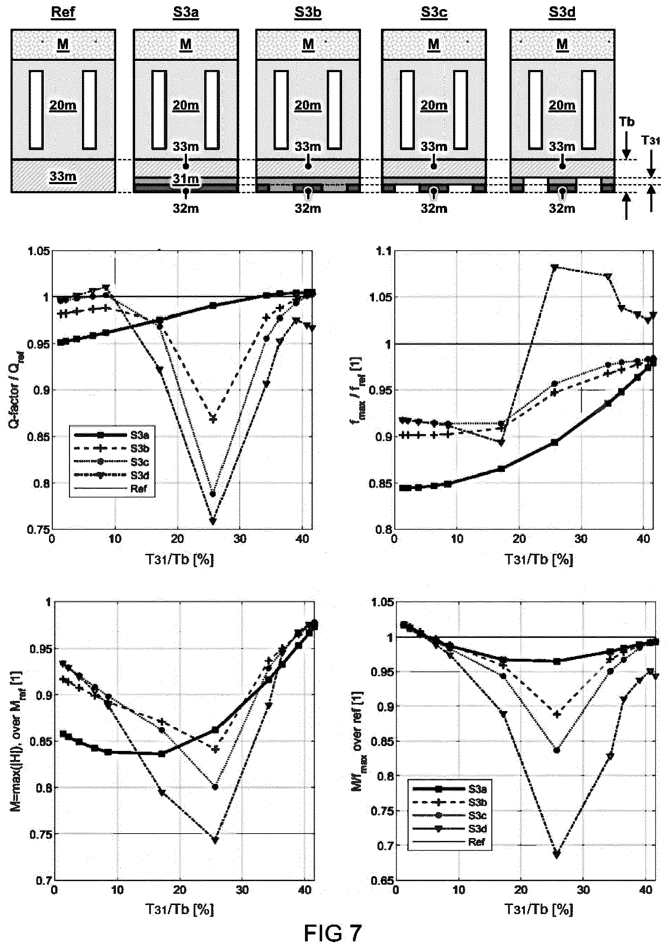

FIGs 6 and 7 illustrate simulations for various structures and thicknesses of sublayers in the backing layer 30. The potential for controlling various parameters of acoustic devices by adaptation of the backing layer is illustrated here using numerical Finite Element Method (FEM) simulations performed in Comsol. Various possible embodiments were simulated and compared to a reference monolithic backing design. The frequency response was simulated, allowing evaluation of parameters including the center frequency, bandwidth, and efficiency. Cross-section views of the various geometries are illustrated above the corresponding graphs.

Without being bound by theory, it will be understood that the quality factor or Q factor is a dimensionless parameter that describes how underdamped an oscillator or resonator is. In one definition, the Q factor is the ratio of the initial energy stored in the resonator to the energy lost in one radian of the cycle of oscillation. In another definition, the Q factor is the ratio of a resonator's centre frequency to its bandwidth when subject to an oscillating driving force. These two definitions give numerically similar, but not identical, results. In any case, it is understood that (sinusoidally) driven resonators having higher Q factors resonate with greater amplitudes (at the resonant frequency) but have a smaller range of frequencies around that frequency for which they resonate, i.e. smaller bandwidth; and (sinusoidally) driven resonators having lower Q factors resonate with lesser amplitudes (at the resonant frequency) but have a higher range of frequencies around that frequency for which they resonate, i.e. larger bandwidth.

FIG 6 illustrates bi-material designs where the relatively high density (heavy) material 32m is steel and where the total backing thickness "Tb" is kept constant at 14pm. Similar results (not shown) were obtained using lead as the heavy material 32m. All quantities are normalized to the performance of the reference design ("Ref"). As illustrated in the legend, the various lines correspond to the different designs "S2a" - "S2c". Along the X-axis the thickness budget allocated to the polyimide PI sublayer / foil is increased, up to 100% at the right, where all designs degenerate into the reference design (monolithic PI). The Y-axis respectively indicates: "Q-factor/Q_{ref}", i.e. the relative value of the Q-factor, wherein a lower Q-factor corresponds to a higher bandwidth; "fₘₐₓ/f_{ref}", i.e. the frequency fₘₐₓ where the signal is maximal, compared to the reference design; "M=max | H | over M_{ref}", i.e. a measure of efficiency calculated as the maximum amplitude of the transfer function H of acoustic waves in the medium compared to the reference design; and "M/fₘₐₓ over ref", i.e. the same, but divided by the fₘₐₓ and compared to the reference design, is a measure of efficiency combining the resonance frequency shift.

As illustrated, with the "S2a" design, a Q-factor reduction of up to 15% can be achieved (top-left), with a 22% reduction in resonance frequency (top-right) when the PI is fully replaced by steel (T33/Tb=0%). The designs "S2b" and "S2c" using patterning are lower, but these designs provide a lower bending rigidity. In all cases, the effect on the radiation efficiency "M" is relatively small, or even increase compared to the reference. A stronger effect can be achieved when the material 32m is more dense, as tested with lead. By optimizing the patterning design further performance can be achieved while keeping the bending stiffness low. In comparative simulation (not shown), the monolithic backing thickness "Tb" was increased from 14pm to 50pm, where it was observed that the resonance frequency is reduced by 21% with only marginal improvement in the bandwidth Q -2%. So, a similar resonance frequency reduction is achievable using the bi-material backing without thickness increase and with the benefit of a bandwidth increase. In yet further simulations (not shown), it was observed that with a backing layer thickness "Tb" of 50pm, the bi-material designs allow 40% resonance frequency reduction and 20-30% Q-factor decrease (tested with lead).

FIG 7 illustrate tri-material backing designs. The total thickness is kept at its reference value "Tb"=14pm, 8µm are allocated the material layer 33m (here PI) and the rest is partitioned between the soft material (vanishing on the left side of the plot) and the relatively high density material (steel, vanishing on the right side). The properties of the soft material 31m, are: density p=1600 kg/m³; Young's modulus E = 30 MPa; Poisson ratio v = 0.495; attenuation α = 0.94 dB/λ. For example, this may correspond to a relatively flexible polymeric material such as polyurethane. On the right side of the plot, the dense material is thin (30% of Tb= 4.2 pm): not much mass is added, leading to a limited decrease in resonance frequency (<10%). Yet a significant bandwidth can be achieved (Q-factor reduction 10-20%). It is associated with a reduction of efficiency that is more or less significant depending on the design. Compared to bi-material designs, a larger Q-factor reduction can be achieved at constant backing thickness. It may be noted that there is a significant difference between the designs "S3b", "S3c", "S3d",which have various degrees of patterning in the backing layer, and the unpatterned backing layer of design "S3a". Without being bound by theory, it is noted that without the patterning, the expansion or contraction of the flexible material 31m may be limited. For example, if the Poisson ratio is close to 0.5, expansion of the material in one direction may require contraction in a transverse direction - for which there is limited possibility in an unpatterned intermediate layer. In conclusion, the simulations demonstrate the ability of bi-material backings to decrease the resonance frequency and Q-factor at constant backing thickness and radiation efficiency; and the ability of tri-material backings to decrease the Q-factor even further. For both, this can be achieved while reducing the bending stiffness compared to monolithic backing.

Among other advantages, the present teachings allow for the lowering of the resonance frequency of the transducer without increasing the thickness of the piezoelectric layer in the transducer device. As described herein mass can be added to the piezoelectric transducer - essentially making it a weakly damped harmonic oscillator. This may reduce the center frequency for a constant thickness, e.g. maintain the mechanical flexibility of the transducer array. Alternatively, or additionally, the present teachings may allow for increasing/maximizing the bandwidth (and thus axial resolution) without increasing the backing thickness. This can also be done while maintaining the mechanical flexibility of the transducer array. Advantageously, the present teachings may obtain large bandwidths without resorting to the excitation of higher order longitudinal modes. Therefore, no transducer efficiency is sacrificed.

Some aspects of the present teachings can be embodied as a method of manufacturing the acoustic device as described herein. In one embodiment, one or more performance parameters (such as the Q-Factor, center frequency fₘₐₓ, efficiency "M") of acoustic transducers in the sheet (100) are calculated as function of a variable layer thickness and/or variable pattern of at least one of multiple sublayers 31,32,33 forming the backing layer 30. In another or further embodiment, the sheet is manufactured in accordance with a layer thickness and/or pattern of the at least one of multiple sublayers 31,32,33 which is selected based on the calculated one or more performance parameters. In another or further embodiment, the calculation is constrained by a maximum thickness of the backing layer and/or minimum flexibility of the sheet.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An acoustic device (1000) comprising a sheet (100) having
a contact surface (11) for contacting a medium (M) and transceiving acoustic signals (A) via the contact surface (11);
a piezoelectric layer (20) comprising piezoelectric material (20m) configured to transduce the acoustic signals (A) via the contact surface (11); and
a backing layer (30) acoustically interconnected to a backside (Sb) of the piezoelectric layer (20), opposite the contact surface (11),
wherein the backing layer (30) comprises
a first sublayer (31) comprising a first material (31m) having a Young's modulus less than that of the piezoelectric material (20m); and
a second sublayer (32) comprising a second material (32m) having a density higher than that of the first material (31m);
wherein at least some of the first material (31m) is arranged between the second material (32m) and the piezoelectric material (20m).

2. The acoustic device (1000) according to claim 1, wherein the sheet is a flexible sheet, wherein thicknesses and materials of all layers forming the sheet (100) are configured to allow bending of the sheet at, or below, a predetermined radius of less than ten centimeter without breaking and/or losing essential functionality.

3. The acoustic device (1000) according to any of the preceding claims, wherein the piezoelectric layer (20) between the front layer (10) and the backing layer (30) has a first thickness (Tp), wherein the backing layer (Tb) between the piezoelectric layer (20) and a backside (Sb) of the device (1000) has a second thickness (Tb), wherein the second thickness (Tb) is smaller than the first thickness (Tp) by at least a factor two.

4. The acoustic device (1000) according to any of the preceding claims, wherein the sheet (100) comprises a front layer (10), between the piezoelectric layer (20) and the front side (Sf) of the device including the contact surface (11), wherein the front layer (10) has a third thickness (Tf) that is smaller than the second thickness (Tb) of the backing layer (30).

5. The acoustic device (1000) according to any of the preceding claims, wherein the piezoelectric layer (20) is a structured piezoelectric layer with subdivided elements of piezoelectric material (20m) forming respective acoustic transducers.

6. The acoustic device (1000) according to any of the preceding claims, wherein the piezoelectric layer (20) comprises piezoelectric material (Mp) forming an array of pillars (22) configured to transceive acoustic signals (A) via a frontside (Sf) of the device; and least one of a backside piezoelectric layer (23) or a frontside piezoelectric layer (21), wherein the array of pillars (22) is integrally formed with the backside piezoelectric layer (23) and/or the frontside piezoelectric layer (21)

7. The acoustic device (1000) according to any of the preceding claims, wherein the second sublayer (32) comprises subdivided areas of the second material (32m).

8. The acoustic device (1000) according to any of the preceding claims, wherein the first sublayer (31) comprises subdivided areas of the first material (31m).

9. The acoustic device (1000) according to any of the preceding claims, wherein at least one the first sublayer (31) and the second sublayer (32) is subdivided according to a pattern wherein more material (31m,32m) of the respective sublayer (31,32) is provided at positions corresponding to positions of the piezoelectric layer (20) where respective transducers are formed.

10. The acoustic device (1000) according to any of the preceding claims, wherein the second sublayer (32) comprises isolated areas of the second material (32m) without material there between.

11. The acoustic device (1000) according to any of the preceding claims, wherein the first sublayer (31) comprises isolated areas of the first material (31m) without material there between.

12. The acoustic device (1000) according to any of the preceding claims, wherein the piezoelectric layer (20) comprises a polymer based piezoelectric material (20m); the first material (31m) comprises a polymeric and/or organic material; and the second material (32m) comprises a metal and/or ceramic material.

13. The acoustic device (1000) according to any of the preceding claims, wherein the sheet (100) comprises a two-dimensional array of acoustic transducers, formed by parts of the piezoelectric layer (20) sandwiched between respective electrode layers (15,35).

14. A method of manufacturing the acoustic device (1000) according to any of the preceding claims, the method comprising
calculating one or more performance parameters (Q-factor, fₘₐₓ, M) of acoustic transducers in the sheet (100) as function of a variable layer thickness and/or pattern of at least one of multiple sublayers (31,32,33) forming the backing layer (30); and
manufacturing the sheet (100) in according with a layer thickness and/or pattern of the at least one of multiple sublayers (31,32,33) based on the calculated one or more performance parameters (Q-factor, fₘₐₓ, M).

15. The method according to the preceding claim, wherein the calculation is constrained by a maximum thickness of the backing layer (30) and/or minimum flexibility of the sheet (100).
